# EUROPEAN PATENT SPECIFICATION

(11) **EP 4 346 921 B1**
(45) Date of publication and mention of the grant of the patent: **02.07.2025**
(21) Application number: 22728297.7
(22) Date of filing: 30.05.2022
(51) Int. Cl.: A61L 9/14, A61L 9/20, B05B 17/00, B05B 17/06, A62B 18/00, A62B 18/02

(54) **WEARABLE DEVICE FOR THE TREATMENT OF BREATHING AIR**
TRAGBARE VORRICHTUNG ZUR BEHANDLUNG VON ATEMLUFT
DISPOSITIF PORTABLE POUR LE TRAITEMENT DE L'AIR RESPIRATOIRE

(30) Priority: 04.06.2021 IT 202100014579
(43) Date of publication of application: 10.04.2024
(73) Proprietor: D'Ambrosio, Gerardo, 50038 Scarperia e San Piero (FI) (IT)
(72) Inventor: D'Ambrosio, Gerardo, 50038 Scarperia e San Piero (FI) (IT)
(74) Representative: Soldatini, Andrea
(86) International application number: PCT/IB2022/055056
(87) International publication number: WO 2022/254314

(56) References cited:
- WO-A1-2021/206061
- DE-U1- 202020 004 189
- JP-B1- 6 954 702
- US-A1- 2017 361 133
- US-A1- 2021 339 058

## Description

### Technical field of the invention

The present invention relates to a device configured to be worn on the head of a user and provided with breathing air purification functionality. In particular, the invention relates to such a device in which the purification functionality is ensured by leaving the user's face substantially free.

### Background of the invention

Devices of the type indicated above are known, which in a structure that can be said to be generically annular provide for conduits evolving at the sides or at the base of the user's head in such a way as to feed a flow of purified air towards the nose-buccal area of the user for inhalation by the user himself. To this end, the conduits are associated with, and mutually connected, both mechanically and pneumatically, to an air treatment unit adapted to be arranged in the rear region of the user's head and which comprises pneumatic flow propulsion means with impeller member, and air treatment means adapted to perform the function of purifying the pneumatic flow. Among the known solutions, some involve leaving the user's face substantially open and free, with the conduits terminating in openings that can be positioned at the sides of the aforesaid nose-buccal region. Among them, the devices shown for example in documents WO2015/140776, US10821255, CN106669056, US2017/36113 and DE202020004189 are worth mentioning.

A solution of this type makes the device reasonably effective in ensuring a certain amount of protection from atmospheric pollution (making sure that the user can inhale a higher quantity of treated/filtered air than normal atmospheric air and/or of the external environment), while at the same time safeguarding the relational capacities ensured by the face left clear, and as well as obviously achieving a good wearing comfort, without discomfort that prevents the continuous or prolonged use thereof.

A functionality of suction and consequent treatment/filtering of the exhalation air may also be provided, but this tends to be provided in the context of devices that mask the nose-buccal area, such as the device that is the subject-matter of document WO2013/082650. Thus, in the latter case, these systems are hardly tolerable except in special circumstances and for limited periods of time.

In general, the forced ventilation system adopted in these known devices is in any case such as to create an open circuit, in which a substantial part of purified air is dissipated into the environment, and the filtration system has the burden of continuously producing new treated air starting from completely untreated ambient air.

In addition to this, the treatment and/or filtering unit adopted in such systems, although it may present acceptable performance in certain cases, is usually subject to periodic replacement operations of filters, cartridges or similar, or in any case force the user to frequent, laborious but also and above all very expensive maintenance operations. Some of the known devices are in any case relatively bulky, heavy and therefore not likely to encourage their use.

### Summary of the invention

Taking this state of the art into account, the Applicant has now developed a wearable breathing air purification device, of the type configured to be worn on the head, which, unlike to what is made available by the prior art, simultaneously achieves all of the following purposes:
- high filtration or purification efficiency with reduced energy consumption and with a low-maintenance filtration/treatment system;
- wearing comfort by leaving the nose-buccal area free, with consequent possibility of continuous use without compromising social and relational activity, and without the risks of hypoxia induced by mask systems of any kind;
- efficacy both with respect to pollutants and with respect to infectious agents;
- compactness, lightness and ergonomics.

These and other ancillary objects are achieved by the wearable breathing air treatment device, the essential characteristics of which are defined by the first of the appended claims. Other important additional features are the subject matter of the dependent claims.

### Brief description of the figures

The features and advantages of the wearable breathing air treatment device according to the present invention will become clearer from the following description of the embodiments thereof, made by way of example and not limitation, with reference to the accompanying drawings in which:
- Figure 1 is an axonometric view of a wearable device according to the present invention, in a first embodiment;
- Figure 2 shows schematically the device of Figure 1 worn on the head of a user;

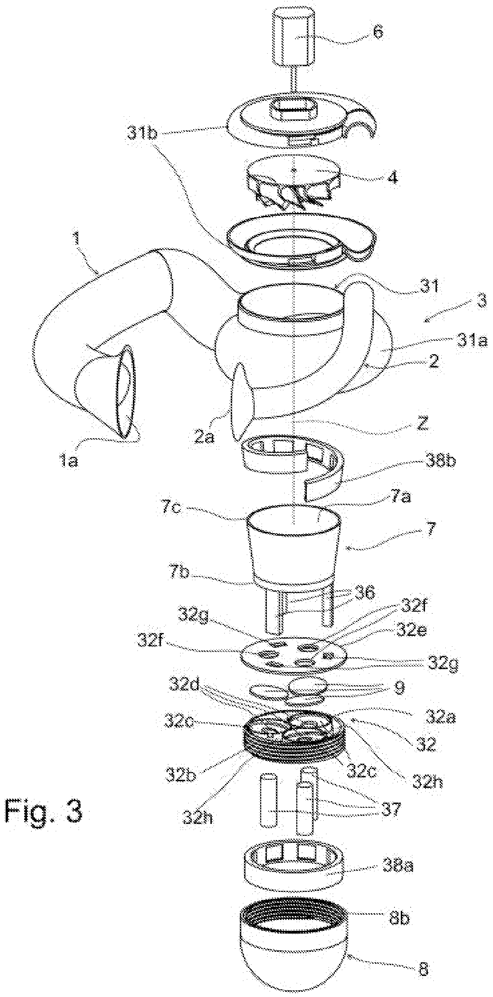

- Figure 3 is, again represented in axonometry, an exploded view of the device in the first embodiment of the previous figures;
- Figure 4 is a top view of the device of the previous figures, with parts split at different levels to highlight the pneumatic circuitry;
- Figures 5 and 6 are sectional views of the device, with parts omitted of Figure 6, according to section planes V-V and VI-VI of Figure 4, respectively;
- Figure 7 is a cutaway axonometric view of an air treatment unit of the device in the previous figures;
- Figure 8 is an axonometric view of a wearable device according to the present invention, in a second embodiment;
- Figure 9 is, again represented in axonometry, an exploded view of the device in the second embodiment of Figure 8;
- Figure 10 is a sectional view of the device of Figures 8 and 9, conducted on the prevailing development plane of the device; and
- Figure 11 is a sectional view of the device of the second embodiment, according to the section plane XI-XI of Figure 10.

### Detailed description of the invention

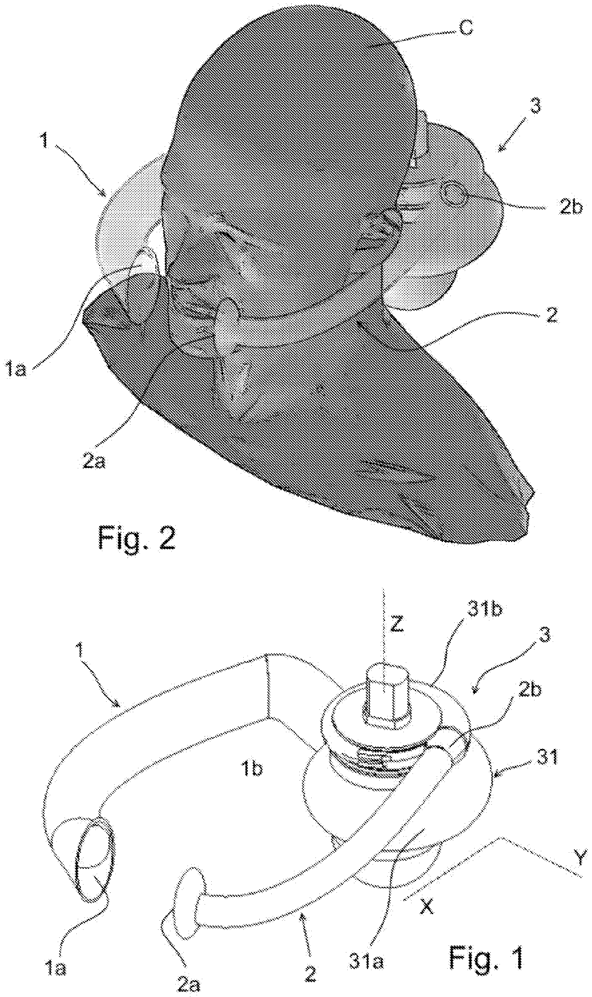

With reference to said figures, a device according to the invention has a substantially annular structure configured to be worn on the head C of a user (Figure 2) while leaving the face substantially free.

The device comprises (numerical indices referring to both embodiments) at least one air suction conduit 1, 101, evolving between a suction inlet 1a, 101a of air to be treated containing suspended pollutant particles, which when the device is worn is arranged near the nose-buccal region of the user, and an air outlet 1b, 101b which is arranged in the near region of the user's head.

At least one ejection conduit 2, 102 then evolves between a treated air ejection outlet 2a, 102a, in turn arranged near the nose-buccal region (tendentially on the opposite side with respect to that where the suction inlet 1a, 101a faces) and in line with it, and an inlet 2b, 102b adapted to be arranged in the rear region of the head. Preferably, the section of the ejection conduit, at least at the end with the outlet gap, is smaller than the corresponding section (end with the suction inlet gap) of the suction conduit, in order to facilitate the priming of air.

The two conduits 1, 2, 101, 102 are mechanically and pneumatically (with the outlet of the suction conduit and with the inlet of the ejection conduit, respectively) joined to an air treatment unit 3, 103, which is therefore in turn adapted to be arranged in the rear region of the head.

The treatment unit 3, 103 comprises pneumatic flow propulsion means, for promoting the pneumatic flow from the suction conduit to the ejection conduit, practically realising a semi-closed loop circuit whereby air is sucked in from the naso-buccal region, passes through the treatment unit, and is ejected back into the aforesaid region. The only opening is represented by the volume of separation between the outlet-inlet of the respective conduits in the nose-buccal region of the user, a volume in which there is a prevalence of treated/healthy air available for the user's inspiration. However, due to the way in which the inlet-outlet gaps are positioned, it is a substantial part of the treated air emitted by the ejection conduit that is gradually sucked back by the suction conduit, and in this sense one can speak of a circuit approximating a semi-closed condition.

The treatment unit then comprises the actual treatment/filtering system 5, 105, which will be discussed in more detail shortly, and an energizing system such as a rechargeable battery-powered micromotor 6, 16 to move the impeller member and to energetically power the treatment/filtering system. The wirings, switches and in general what is generally required to ensure the operation of the simple electrical components adopted in the device are not represented, as they are of obvious nature and obviously implementable. For example, and in particular, obvious means will be provided for the regulation or partialisation of the flow rate and/or of the speed of the air flow within a preestablished intervention to adapt the device to its own needs, acting on the operation of the motor and/or on the section of the conduits.

Again according to the invention, the treatment/filtering system comprises a tubular chamber 7, 107, for example but not necessarily conical or more properly truncated conical, defining an inner lateral surface 7a, 107a, evolving according to an axis Z, Y' between a first end 7b, 107b, having a smaller diameter and in pneumatic communication with the outlet of the suction conduit, and a second end 7c, 107c, having a larger diameter, in pneumatic communication with said inlet of the ejection conduit, and therefore influenced by the circulation promoted by the pneumatic flow propulsion means. Pneumatic flow directing means adapted to promote a swirling circulation within the chamber itself are also associated with the chamber.

According to a preferred solution, but which does not exclude other possible solutions, the propulsion means and the means for directing the pneumatic flow are made by the same component, an impeller member 4, 104 which is advantageously arranged coaxially to the chamber intercepting the inside thereof, with rotation axis Z, Y' and configured with adequate blading like a centrifugal impeller.

A water reservoir 8, 108 communicates with the chamber, being placed near the first end 7b, 107b having a smaller diameter. Said reservoir is affected by water nebulizer means 9, 109, e.g. means for stirring the water contained therein, configured to promote an emission of nebulized water entering the chamber through the first end, whereby the nebulized water is invested by the air to be treated entering the chamber through the first end, and by effect of the swirling circulation promoted for example by the impeller, a deposit of water retaining said pollutant particles is formed in adhesion on the inner lateral surface of the chamber and tends to return to the reservoir, from which they can be disposed of periodically, with water supplementation/replacement, thanks to a reversible connection between the reservoir and the rest of the unit.

This being said in general terms, with more specific reference to the embodiment options, and therefore here to the first embodiment and to the relative Figures from 1 to 7, the treatment unit 3 is configured with the axis Z of rotation of the impeller and of development of the chamber that is arranged orthogonally to the plane XY of the general development of the device, i.e. a plane defined by the layout of the conduits and which in the arrangement of use of the device corresponds to a transverse plane of the head. In practice, in this arrangement of use and with the user in an upright position, the axis Z is substantially vertical.

The treatment unit 3 in this case comprises a box-like body 31 for housing and supporting the other components, defined by a lower portion 31a and by an upper portion 31b which are generically disc-shaped, having a larger cross-section for the lower portion, which evolve with polar symmetry with respect to the axis Z. The suction 1 and ejection 2 conduits engage respectively in the lower portion and in the upper portion of the body with a substantially tangential angle.

The lower portion or volute 31a is shut at the base by a pervious diaphragm 32, lying orthogonally to the axis Z and to which we will return later, which thus separates the inside of the box-like body 31 from the underlying reservoir 8, components among which a reversible connection can advantageously, as mentioned, operate, for example with screws 32c-8b with suitable seals (not shown). The lower portion 31a also centrally houses a sleeve 7, for example just conical, which materialises the above-mentioned chamber, so that the second end 7c, in this case upper, is adjacent to the upper portion or volute 31b. Around the sleeve 7 the lower portion 31a develops a lower toroidal channel 33 for distributing air, fed by the suction conduit 1, around the base of the sleeve itself. The latter with the first end 7b thereof is spaced with respect to the diaphragm 32, so as to create a passage 34 of the pneumatic flow between the toroidal channel and the inside of the sleeve or chamber 7.

The upper portion 31b centrally houses the centrifugal impeller 4, which as mentioned is arranged at the second end 7c of the conical sleeve 7, defining around it an upper toroidal channel 35, for the radial distribution of the air and for directing it towards the ejection conduit 2.

Returning to the pervious diaphragm 32, in the illustrated embodiment it has a main septum 32a with a distribution of seats 32c housing respective stirring elements in the form of porous piezoelectric foils 9 with which the aforesaid nebulizer means are advantageously made. In this embodiment solution, the water is fed to the foils, which by vibrating at an appropriate frequency take charge of stirring, and consequent diffusion of nebulized water within the chamber 7 from the first end 7b, advantageously exploiting the phenomenon of capillarity through ducts 37 which rise from the inside of the reservoir and penetrate the septum 32a suitably provided with holes 32d, below the seats 32c. These ducts are, for example, materialised by bodies of known structure, made of porous or spongy material such as cellulose acetate, which form small channels precisely suitable to produce the physical phenomenon of the rise of water by capillarity. Beyond this possible embodiment solution by vibration, which, as mentioned, is advantageous in terms of simplicity and efficiency, the nebulizer means may include other types of expedients adapted to produce an equivalent result in the formation of an aerosol.

The diaphragm 32 is completed by a cover 32e superimposed on the septum 32a and which closes the seats 32c of the piezoelectric foils, bearing at their correspondence a first distribution of holes 32f to allow the passage of nebulized water towards the chamber. The plate-shaped cover 32e also bears a second distribution of holes 32g, which correspond to passages 32h of the septum 32a, within which respective discharge tubes 36 penetrate and extend from the first end 7b of the conical sleeve 7, the first end being folded inwards to form a perimeter shower 7d which has the function of collecting the water mixed with impurities falling along the inner lateral surface 7a, a water that the communication between the shower and the discharge tubes 36 allows to precipitate back into the reservoir.

The device can still be provided with other treatment systems, such as one or more UVC-radiating LED strips, in this case a lower annular strip 38a in the reservoir 8 and an upper helical strip 38b around the sleeve 7, all to add precisely a germicidal action that as we know can be carried out by the aforesaid type of radiation, suitably adjusted.

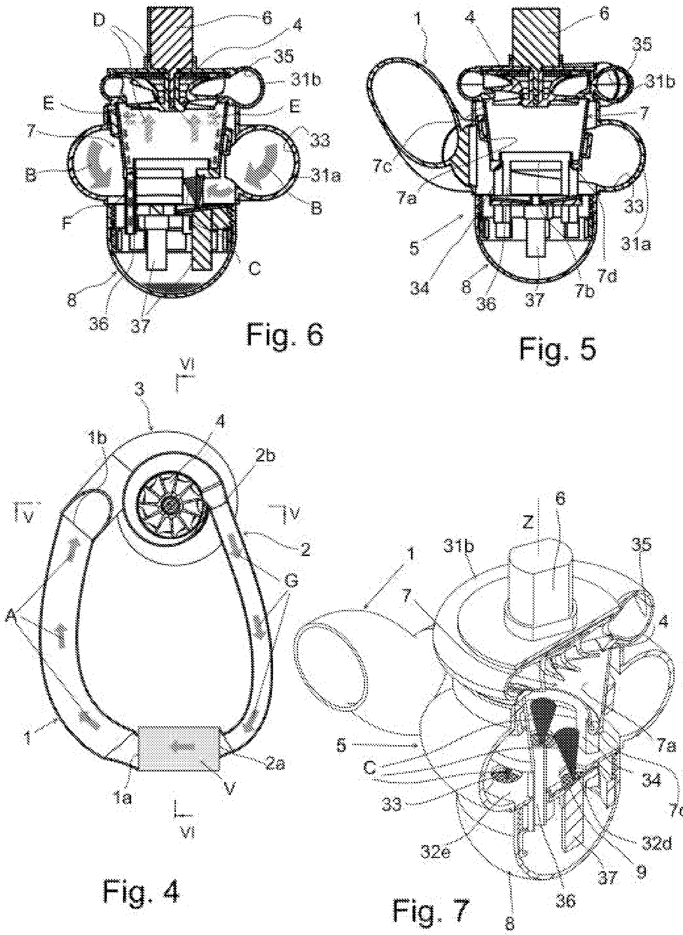

The device described so far therefore operates as follows, with particular reference to Figures 4 and 6 in which the arrows schematise the air and water flows. Once worn on the head and the conduits are in place, the outlet gap 2a of the ejection conduit 2 and the inlet gap 1a of the suction conduit 1 are located in the naso-buccal region, delimiting an exchange volume V which is influenced by the pneumatic action of the device and which represents precisely an air exchange volume, as it faces the breathing cavities, invested by the user's inhalation and exhalation activity.

During inspiration, the incoming flow taken from the volume V is replenished by air coming from the surrounding environment. During the exhalation phase, the breath joins the flow entering the device. In this way, the device repeatedly processes and purifies a large mass of air, comprising the same exhaled air, increasing the degree of cleanliness.

Following the path of the air to be treated, which comprises pollutants in the form of particulate matter, ash, volatile substances in suspension, etc., which path as said is promoted by the impeller 4, the air then enters the suction conduit 1 through the inlet or suction inlet gap 1a (arrows A of Figure 4) and reaches the lower toroidal channel 33 of the lower portion, distributing itself at the base of the sleeve 7 and being drawn axially into it through the first end 7b thereof (arrows B of Figure 6). Here the air invests the emission of nebulized water that rises from the diaphragm 32 due to the vibration of the piezoelectric foils 9 (schematised by the conical regions C of Figure 6 and Figure 7). The action of the vortex induced by the impeller by suction on the mixture of air and polluting particles/dust favours the aggregation of these particles in suspension with the micro-droplets of water produced by the foils. This mixture is drawn towards the impeller within the chamber defined by the sleeve and along the axis thereof by a swirling circulation (the axial component of which is represented by the arrows D of Figure 6).

By approaching the second end 7c of the chamber 7, the swirling flow takes on a strongly centrifugal component (arrows E of the same Figure 6). The tangential speed course of the mixture grows as it approaches the impeller mouth, consequently increasing the centrifugal effect. Near the mouth of the impeller 4, the droplets and the incorporated material thus tend to settle on the inner lateral surface 7a, while the air enters the compartments of the impeller blading.

Due to the diverging diameter shape of the surface, and to the pressure gradient generated in the chamber, this substantially liquid deposit tends to descend towards the first end 7b and from there by gathering in the shower 7d and through the discharge tubes 36 (arrows F of Figure 6) is deposited at the bottom of the reservoir 8 where the polluting component removed from the pneumatic flow also accumulates. Impurities gathered in the reservoir, where they settle at the bottom, can be removed by unscrewing the reservoir, an operation with which consumed water will also be gradually replenished. The treated air is distributed downstream of the impeller into the upper toroidal channel 35 and from it into the ejection conduit 2 (arrows G of Figure 4) to join again the exchange volume V outside the device, laterally investing the mouth and the nose of the user, who will benefit from the inhalation of air that is for the most part treated and purified.

The device thus ensures a continuous inflow of air, which eliminates all problems related to hypoxia phenomena induced by traditional filter masks. The continuous circular flow allows large amounts of air to be (re)treated several times (the percentage of air already treated can be as high as 85-90% of the air being gradually sucked in), thus allowing to increase the level of air purification. The configuration of the device allows the front of the face to be left unobstructed, guaranteeing an absolute benefit in relational/social terms. By appropriately sizing the impeller and the passage sections of the pneumatic circuit, it will be possible to ensure a redundant volume supply of emitted and treated air (up to 60 litres air/min) and a high flow speed (e.g. around 10 m/s), which requirements ensure the highest quality of the air that the user inhales.

Appropriate aromatic or even medicinal substances can be dissolved in the water reservoir, which once nebulized are breathed in by the user, bringing further benefits. More generally speaking, it should be pointed out that, when mentioning water, in the context of the present disclosure, it was meant to indicate the simplest liquid that tends to be apt for use, without, however, ruling out the possibility that other liquid substances may be used to the extent that they are suitable for ensuring the operation of the device.

Evidently, the device does not require filters that need being replaced periodically, once they have reached their saturation. The use of water (preferably distilled) to operate the filtration has no environmental impact and requires simply replenishing it after a certain number of hours, depending on the conditions of use.

The conduits are made wholly or partially of material or technology that is suitable to provide them with adequate resilience, possibly even with a certain elasticity, so as to ensure on the one hand the deformability necessary for wearing, and on the other hand adaptability to the physiognomy of the user and contextually a wearing that is stable and at the same time comfortable. However, it is not excluded that the device can be equipped with accessories or aids to further increase comfort and stability, e.g. padding, head support laces and the like. Likewise, the conceptual configuration of the device could be integrated into more wraparound structures (helmets or similar).

The construction of the treatment unit can easily be made with dismountable components, to facilitate washing with running water and suitable detergents to remove any impurities that may remain in the conduits, before subsequent use. The adoption of UVC LED technology enhances sterilisation functionalities to combat microorganisms and viruses that are potentially dangerous to human health.

The power supply battery for the supply of adequate autonomy to the motor, to the nebulizer means and to any UVC LED means can be rechargeable according to standard technologies, for example by means of a USB connector. The motor can be a simple 6V micromotor adapted to generate, for example, an impeller rotation speed of 12/13000 rpm. Depending on the embodiment, other technologies or speed ranges may be more appropriate.

The person skilled in the art will also have no difficulty in implementing any sensors capable of monitoring in real time the quality of the air treated, the reservoir filling, and other control parameters of the device. These sensors may eventually dialogue, by equipping the device with appropriate electronic and communication components, with a dedicated application developed for smartphones, so as to manage the device via the application itself, as well as monitor personal parameters relating, for example, to respiratory coefficients. The sharing of certain collected data via network, subject to authorisation by users, may also allow real-time air quality mapping to be carried out in a defined region.

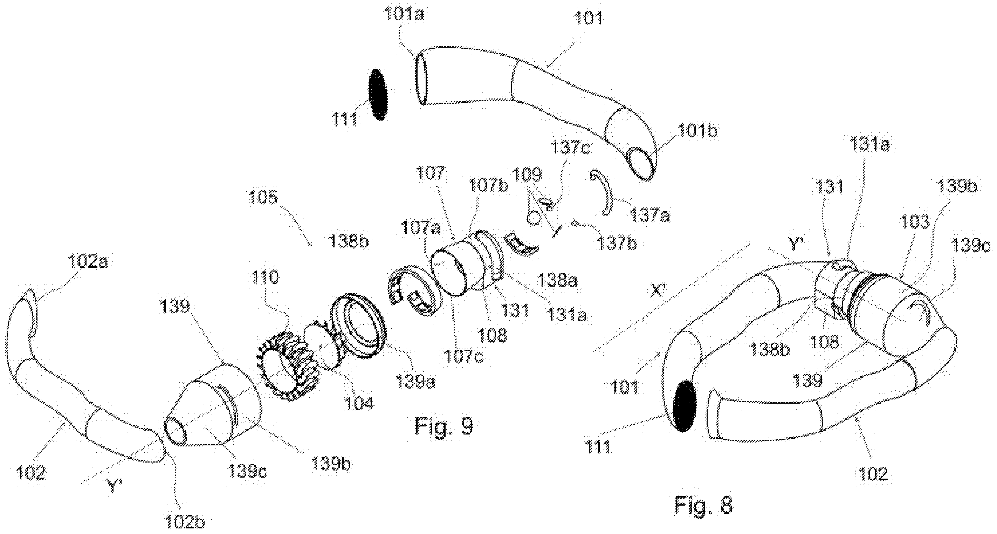

Above and beyond what has been described for the first embodiment, it is clear that the invention can be reduced to practice with different construction solutions. Among these, in a second embodiment, which is the specific subject of Figures 8 to 11 to which reference is made below, and in the treatment unit 103 the common axis of the impeller and of the chamber, indicated in this case with Y', is lying on the plane X'Y' of general development of the device as already defined above, or in any case parallel to it. In practice, in the arrangement of use and with the user in an upright position, the axis Y' is substantially horizontal and oriented in the direction of laterality.

The first end having a smaller diameter 107b and the second end having a larger diameter 107c of the chamber 107 (defining the inner surface 107a) are thus oriented, again with the device worn, on the respective sides of the user's head, and the suction 101 and ejection 102 conduits are joined there with a substantially (co)axial angle. Consequently, the pneumatic flow develops with the main component remaining fundamentally axial.

This different configuration gives rise to a number of different construction options of this embodiment, described below remaining in the context of a construction that envisages a centrifugal impeller member to promote and direct the flow (although this construction expedient is not in principle the only one possible also for the embodiment now considered).

With regard to the liquid stirring/feeding functionality, in this case, between the outlet 101b of the suction channel and the first end 107b of the chamber 107, it may be provided for a cylindrical tubular manifold 131 for entering the chamber, having an internal diameter substantially corresponding to that of the chamber 107 at the first end 107b. On the inner surface of the manifold 131 there are defined seats 132 which house respective piezoelectric foils 109, to which water is fed by capillary ducts 137a, 137b, 137c which extend between respective seats 132 and a reservoir 108 herein configured as a radial expansion of the manifold in the lower area (i.e. which is arranged below when the device is worn). To this end, the ducts in this case have different developments, a first duct 137a feeding liquid to the foil furthest from the reservoir having in particular an elongated and arched development according to the circumference of the manifold on which an appropriate rib 131a is provided for the purpose, and two other ducts 137b, 137c for conveyance to foils closest to the reservoir and with substantially radial development.

The lower radial expansion, which as mentioned forms the reservoir 108, can advantageously have an alveolus 108a at the bottom in which the collection of impurities is favoured.

Turning to the outlet area of the purified pneumatic flow, an outlet manifold 139 from the chamber is arranged between the second end 107c of the chamber and the inlet 102b of the ejection conduit 102, and comprises, in succession following the axial course of the pneumatic flow a cylindrical segment 139b immediately downstream of the chamber, with a diameter corresponding to that of the latter at the second end, and a conical segment 139c which narrows the passage section up to that of the ejection conduit.

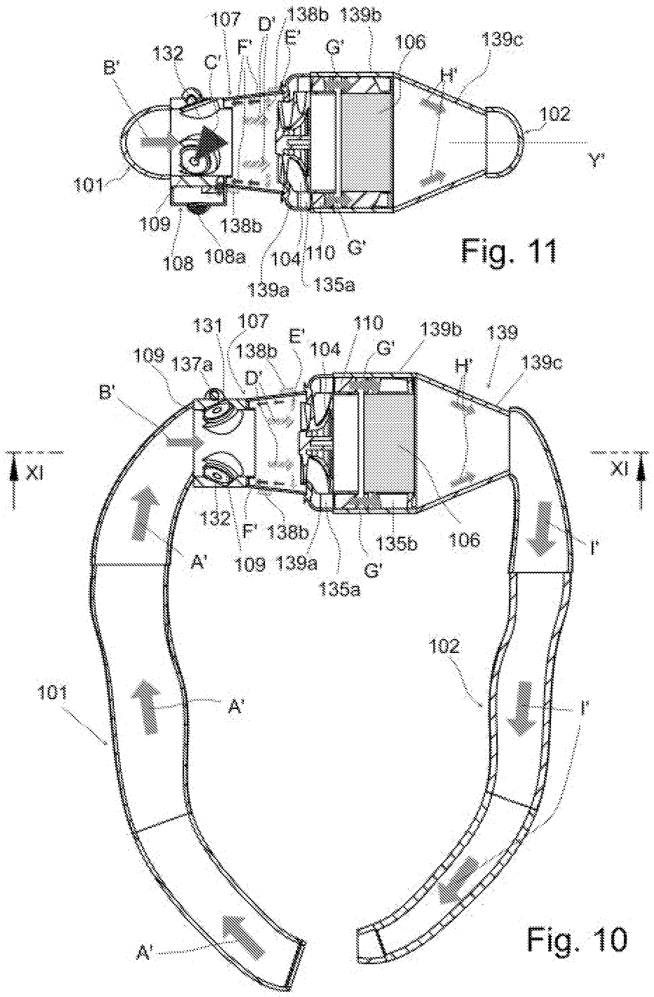

More specifically, the cylindrical segment 139b is joined to the chamber 107 by means of a connecting cup 139c in which the centrifugal impeller 104 is centrally housed and which peripherally defines a toroidal channel 135a for distributing and directing the outgoing air. Downstream of the impeller, and thus in the cylindrical segment 139b as such, there is a rotor 110 driven in rotation together with the impeller 104 and evidently coaxially with it. The rotor 110 is configured with an axial blading to further promote the flow from the toroidal channel 135a in an axial direction, and to inject it into an annular cavity 135b, which again within the cylindrical segment 139b surrounds a housing compartment for the motor 106 (schematically shown in Figures 10 and 11 only). A stator element, not represented here, is generally useful for conveying the circulation in the axial direction between impeller and rotor, according to what is obvious to the person skilled in the art.

Finally, the conical segment 139c has the task of conveying the pneumatic flow up to the inlet 102b of the ejection conduit 102. UVC-radiating LED strips are or can be provided in this embodiment as well, in the form of a first arc strip 138a in the reservoir 8 and an upper helical strip 138b around sleeve 107. Finally, the drawings of this embodiment exemplify an evolution of the conduits which envisages, as already provided above in general and evidently also usable in the context of the first embodiment, at least resilient, deformable or articulated central sections to improve adaptation to the user's head, and a mesh filter 111 which can intercept the inlet 101a of the suction conduit is also represented.

Following also in this case the path of the air to be treated, it then enters the suction conduit 101 arrows A' of Figure 10) and arrives directly in an axial direction at the base of the sleeve 107, being drawn axially into it through the first end 107b thereof (arrows B' of Figure 10 and Figure 11). Here, the air invests the water nebulized by the piezoelectric foils 109 (conical region C' in Figure 11). Again, it is the vorticity induced by the impeller that favours the aggregation of the suspended particles with the micro-droplets of water produced by the foils. This mixture is drawn towards the impeller within the chamber and along the axis thereof by a swirling circulation (the axial component of which is represented by the arrows D' of Figure 11).

Towards the second end 107c of the chamber 107, the centrifugal component (arrows E') of the flow pushes the droplets and the incorporated material so as to settle on the inner lateral surface 107a, while air enters the compartments of the impeller. It is mainly the pressure gradient generated in the diverging chamber that in this case promotes drawing the liquid deposit towards the first end 107b and the accumulation in the reservoir 108 (arrows F').

The treated air is distributed downstream of the impeller in the toroidal channel 135a and from it through the blading of the axial rotor 110 into the cavity 135b of the cylindrical segment of the manifold 139, again with axial direction indicated by the arrows G', and is then conveyed into the conical segment 139c (arrows H') and then enters the ejection conduit 102 (arrows I').

This second embodiment also evidently does not exhaust the construction options according to which the present invention can be practically implemented. As much it has been described so far with reference to preferred embodiments, it is therefore to be understood that other embodiments may exist within the scope of protection of the attached claims.

## Claims

1. A wearable air treatment device having a generically annular structure configured to be worn on the head (C) of a user while leaving the face of the user substantially unobstructed, the device comprising:
- at least one air suction conduit (1), evolving between a suction inlet (1a) of air to be treated containing suspended pollutant particles, adapted to be arranged near a nose-buccal region of the user, and an outlet (1b) of air adapted to be arranged in a rear region of the user's head (C);
- at least one air ejection conduit (2) evolving between a treated air ejection outlet (2a) adapted to be arranged near the nose-buccal region of the user and an inlet (2b) adapted to be arranged in the rear region of the user's head (C);
- a air treatment unit (3) connected to said air suction and air ejection conduits (1, 2) and in pneumatic communication therewith between said outlet (1b) of said air suction conduit (1) and said inlet (2b) of said air ejection conduit (2), said air treatment unit (3) being adapted to be arranged in the rear region of the user's head (C) and comprising: pneumatic flow propulsion means from said air suction conduit (1) to said air ejection conduit (2); air treatment means (5) configured to receive air to be treated from said outlet (1b) of said air suction conduit (1), and return treated air to said inlet (2b) of said air ejection conduit (2); and energization means (6) of said pneumatic flow propulsion means and said air treatment means (5);
wherein said air treatment means (5) comprise:
- a tubular chamber (7) defining an inner side surface (7a) of the tubular chamber (7) and evolving along an axis (Z) of the tubular chamber (7) between a first end (7b), having a smaller diameter and in pneumatic communication with said outlet (1b) of the air suction conduit (1), and a second end (7c), having a larger diameter, in pneumatic communication with said inlet (2b) of said air ejection conduit (2);
- pneumatic flow directing means configured to promote a swirling circulation within said tubular chamber (7);
- at least one water reservoir (8) communicating with said tubular chamber (7) and placed near said first end (7b);
- nebulizer means (9) associated with said water reservoir (8), configured to promote an emission of nebulized water entering said tubular chamber (7) through said first end (7b),
whereby:
said emission of nebulized water is invested by the air to be treated entering said tubular chamber (7) from said first end (7b), and by effect of said swirling circulation a deposit of water retaining said pollutant particles is formed in adhesion on said inner lateral surface (7a) of said tubular chamber (7) and tends to return to said water reservoir (8).

2. The device according to claim 1, wherein said pneumatic flow propulsion means comprise at least one impeller member (4).

3. The device according to claim 2, wherein said pneumatic flow directing means are formed by said at least one impeller member (4), configured as a centrifugal impeller and coaxially supported within said tubular chamber (7).

4. The device according to any of the previous claims, wherein said tubular chamber (7) has a truncated conical shape.

5. The device according to any of the previous claims, wherein said nebulizer means (9) comprise water agitator means in sad at least one water reservoir (8).

6. The device according to claim 5, wherein said water agitator means comprise one or more piezoelectric elements (9).

7. The device according to claim 6, wherein said one or more piezoelectric elements comprise one or more foils (9) made of porous material, said nebulizer means further comprising capillary ducts (37) extending between said water reservoir (8) and said foils (9) to supply water from said reservoir (8) to said foils (9).

8. The device according to claim 7, wherein said capillary ducts (37) are provided by one or more bodies made of porous or spongy material.

9. The device according to any of the previous claims, comprising one or more UVC radiation LEDs adapted to perform a germicidal action on water at least in said water reservoir (8).

10. The device according to claim 6, comprising at least a first LED strip (38a) arranged at the level of said reservoir and a second helical LED strip (38b) evolving around said chamber (7).

11. The device according to any of the previous claims, wherein said air suction and air ejection conduits (1, 2) comprise one or more resilient, deformable or articulated sections to improve adaptation to the head (C) of the user.

12. The device according to any of the previous claims, wherein said air suction conduit (1) and said air ejection conduit (2) lie substantially over a plane of general development (X, Y) of the device, which in the use position of the device corresponds to a transverse plane of the user's head (C).

13. The device according to claim 12, wherein said tubular chamber (7) is arranged with said chamber axis (Z) orthogonal to said general development plane (X, Y) of the device.

14. The device according to claim 13, when depending from claim 3, wherein said treatment unit (3) comprises a box-like housing body (31) defined by a lower portion (31a) and an upper portion (31b), both generically disc-shaped and evolving with substantially polar symmetry with respect to said chamber axis (Z), said air suction (1) and air ejection (2) conduits joining said box-like housing body (31) respectively at said lower portion (31a) and at said upper portion (31b) according to a substantially tangential angle, said tubular chamber (7) being formed by a sleeve housed in said lower portion (31a) so that said second end (7c) of the tubular chamber (7) is adjacent to said upper portion (31b), said lower portion (31a) being shut at its base by a pervious diaphragm (32) supporting said nebulizer means (9), said pervious diaphragm (32) separating the inside of said box-like housing body (31) from said water reservoir (8), said lower portion (31a) forming a lower toroidal channel (33) for distributing air, fed by the air suction conduit (1), around the base of the sleeve and towards the inside of the tubular chambe (7) through a passage between the first end (7b) of the tubular chamber (7) and said previous diaphragm (32), said upper portion (31b) supporting said energizing means (6), centrally housing said impeller member (4) and forming around it an upper toroidal channel (35) for the radial distribution of the air and feeding it towards the air ejection conduit (2).

15. The device according to claim 14, when dependent on claim 7, wherein said pervious diaphragm (32) provides a distribution of seats (32c) for accommodating said one or more foils (9), and a distribution of holes (32d, 32f, 32g, 32h) for said capillary ducts (37) and for discharge tubes (36) extending from the first end (7b) of the sleeve, said first end (7b) of the sleeve being folded inwards to form a peripheral gutter (7d) adapted to collect water mixed with impurities falling along the inner lateral surface (7a) of the tubular chamber (7).

16. The device according to claim 12, wherein said tubular chamber (7) is arranged with said chamber axis (Z') parallel to, or lying on, said general development plane (X', Y') of the device.

17. The device according to claim 16 when dependent on claim 4, wherein a chamber intake tubular manifold (131) between said outlet (101b) of the suction channel (101) and said first end (107b) of the tubular chamber (107), said chamber intake tubular manifold (131) having an inner diameter substantially corresponding to that of the tubular chamber (107) at the first end (107b), on the inner surface of manifold seats (132) being formed for housing respective piezoelectric foils (109), to which water is fed by said capillary ducts (137, 137b, 137c) extending between respective manifold seats (132) and said water reservoir (108), the latter being configured as a radial expansion of the chamber intake tubular manifold (131) at a lower zone.

18. The device according to claim 17, wherein said radial expansion forming said water reservoir (108) has at its bottom a cavity (108a) assisting the collection of the pollutant particles.

19. The device according to any of claims 16 to 18, wherein a chamber outtake manifold (139) is arranged between the second end (107c) of the tubular chamber and said inlet (102b) of the air ejection conduit (102), and comprises, in succession following the axial course of the pneumatic flow, a cylindrical segment (139b) immediately downstream of the tubular chamber (107), having a diameter corresponding to that of said second end (107c) of the tubular chamber (107), and a conical segment (139c) which narrows the air passage section to that of the air ejection conduit (102), said cylindrical segment (139b) being joined to the tubular chamber (107) by means of a connecting cup (139a) in which said centrifugal impeller member (104) is housed and which peripherally defines a toroidal channel (135a) for distributing and directing the outgoing air, downstream of said impeller member (104), and therefore towards the cylindrical segment (139b), in the latter a rotor (110) being arranged, driven into rotation together with said impeller member (104) and configured with an axial vane to further promote the flow from said toroidal channel (135a) in the axial direction, and to introduce it into an annular gap (135b) which still inside the cylindrical segment (139b) surrounds a housing for said energizing means (106).

20. The device according to any of the previous claims, wherein at least said water reservoir (8) is supported detachably from the rest of the device.

21. The device according to any of the previous claims, wherein said energizing means comprise a motor (6) and rechargeable battery means.

## Patentansprüche

1. Tragbare Luftaufbereitungsvorrichtung mit einer allgemein ringförmigen Struktur, die konfiguriert ist, um an dem Kopf (C) eines Benutzers getragen zu werden, wobei das Gesicht des Benutzers im Wesentlichen frei bleibt, wobei die Vorrichtung Folgendes umfasst:
- mindestens eine Luftansaugleitung (1), die sich zwischen einem Ansaugeinlass (1a) für aufzubereitende, suspendierte Schadstoffpartikel enthaltende Luft, der in der Nähe einer Nasen-Bukkal-Bereichs des Benutzers angeordnet werden kann, und einem Luftauslass (1b) erstreckt, der an einem hinteren Bereich des Kopfes (C) des Benutzers angeordnet werden kann;
- mindestens eine Luftausstoßleitung (2), die sich zwischen einem Ausstoßauslass (2a) für aufbereitete Luft, der in der Nähe des Nasen-Bukkal-Bereichs des Benutzers angeordnet werden kann, und einem Einlass (2b) erstreckt, der am hinteren Bereich des Kopfes (C) des Benutzers angeordnet werden kann;
- eine Luftaufbereitungseinheit (3), die mit der Luftansaugleitung und der Luftausstoßleitung (1, 2) verbunden ist und mit diesen zwischen dem Auslass (1b) der Luftansaugleitung (1) und dem Einlass (2b) der Luftausstoßleitung (2) in pneumatischer Verbindung steht, wobei die Luftaufbereitungseinheit (3) am hinteren Bereich des Kopfes (C) des Benutzers angeordnet werden kann und Folgendes umfasst: pneumatische Strömungsantriebsmittel von der Luftansaugleitung (1) zur Luftausstoßleitung (2); Luftaufbereitungsmittel (5), die konfiguriert sind, um aufzubereitende Luft von dem Auslass (1b) der Luftansaugleitung (1) aufzunehmen und aufbereitete Luft zum Einlass (2b) der Luftausstoßleitung (2) zurückzuführen; und Energieversorgungsmittel (6) für die pneumatischen Strömungsantriebsmittel und die Luftaufbereitungsmittel (5);
wobei die Luftaufbereitungsmittel (5) Folgendes umfassen:
- eine röhrenförmige Kammer (7), die eine innere Seitenfläche (7a) der röhrenförmigen Kammer (7) begrenzt und sich entlang einer Achse (Z) der röhrenförmigen Kammer (7) zwischen einem ersten Ende (7b) mit kleinerem Durchmesser und in pneumatischer Verbindung mit dem Auslass (1b) der Luftansaugleitung (1) und einem zweiten Ende (7c) mit größerem Durchmesser und in pneumatischer Verbindung mit dem Einlass (2b) der Luftausstoßleitung (2) erstreckt;
- pneumatische Strömungsleitmittel, die konfiguriert sind, um eine Wirbelzirkulation innerhalb der röhrenförmigen Kammer (7) auslösen;
- mindestens einen Wasserbehälter (8), der mit der röhrenförmigen Kammer (7) in Verbindung steht und in der Nähe des ersten Endes (7b) angeordnet ist;
- Vernebelungsmittel (9), die zu dem Wasserbehälter (8) gehörig sind und konfiguriert sind, um eine Abgabe von vernebeltem Wasser auszulösen, das durch das erste Ende (7b) in die röhrenförmige Kammer (7) eintritt,
wobei
die Abgabe des vernebelten Wassers durch die aufzubereitende Luft bewirkt wird, die in die röhrenförmige Kammer (7) von dem ersten Ende (7b) her eintritt, und wobei durch die Wirkung der Wirbelzirkulation an der inneren Seitenfläche (7a) der röhrenförmigen Kammer (7) eine anhaftende Ablagerung von Wasser gebildet wird, das die Schadstoffpartikel zurückhält und dazu neigt, in den Wasserbehälter (8) zurückzukehren.

2. Vorrichtung nach Anspruch 1, wobei die pneumatischen Strömungsantriebsmittel mindestens ein Laufradelement (4) umfassen.

3. Vorrichtung nach Anspruch 2, wobei die pneumatischen Strömungsleitmittel durch das mindestens eine Laufradelement (4) gebildet werden, das als Zentrifugallaufrad konfiguriert und koaxial in der röhrenförmigen Kammer (7) gelagert ist.

4. Vorrichtung nach einem der vorstehenden Ansprüche, wobei die röhrenförmige Kammer (7) eine kegelstumpfförmige Form aufweist.

5. Vorrichtung nach einem der vorstehenden Ansprüche, wobei die Vernebelungsmittel (9) Wasserrührmittel in dem mindestens einen Wasserbehälter (8) umfassen.

6. Vorrichtung nach Anspruch 5, wobei die Wasserrührmittel ein oder mehrere piezoelektrische Elemente (9) umfassen.

7. Vorrichtung nach Anspruch 6, wobei das eine oder die mehreren piezoelektrischen Elemente eine oder mehrere Folien (9) aus porösem Material umfassen, wobei die Vernebelungsmittel ferner Kapillarkanäle (37) umfassen, die sich zwischen dem Wasserbehälter (8) und den Folien (9) erstrecken, um den Folien (9) Wasser aus dem Behälter (8) zuzuführen.

8. Vorrichtung nach Anspruch 7, wobei die Kapillarkanäle (37) durch einen oder mehrere Körper aus porösem oder schwammartigem Material bereitgestellt werden.

9. Vorrichtung nach einem der vorstehenden Ansprüche, umfassend eine oder mehrere UVC-Strahlungs-LEDs, die dazu geeignet sind, eine keimtötende Wirkung auf das Wasser zumindest in dem Wasserbehälter (8) auszuüben.

10. Vorrichtung nach Anspruch 6, umfassend mindestens einen ersten LED-Streifen (38a), der auf Höhe des Behälters angeordnet ist, und einen zweiten spiralförmigen LED-Streifen (38b) umfasst, der sich um die Kammer (7) windet.

11. Vorrichtung nach einem der vorstehenden Ansprüche, wobei die Luftansaugleitung und die Luftausstoßleitung (1, 2) einen oder mehrere elastische, verformbare oder gelenkige Abschnitte aufweisen, um die Anpassung an den Kopf (C) des Benutzers zu verbessern.

12. Vorrichtung nach einem der vorstehenden Ansprüche, wobei die Luftansaugleitung (1) und die Luftausstoßleitung (2) im Wesentlichen über einer allgemeinen Erstreckungsebene (X, Y) der Vorrichtung liegen, die in der Gebrauchsposition der Vorrichtung einer Querebene des Kopfes (C) des Benutzers entspricht.

13. Vorrichtung nach Anspruch 12, wobei die röhrenförmige Kammer (7) so angeordnet ist, dass die Kammerachse (Z) im orthogonal zu der allgemeinen Erstreckungsebene (X, Y) der Vorrichtung verläuft.

14. Vorrichtung nach Anspruch 13, sofern dieser von Anspruch 3 abhängt, wobei die Aufbereitungseinheit (3) einen kastenförmigen Gehäusekörper (31) umfasst, der durch einen unteren Abschnitt (31a) und einen oberen Abschnitt (31b) begrenzt ist, die beide im Allgemeinen scheibenförmig sind und sich mit im Wesentlichen polarer Symmetrie in Bezug auf die Kammerachse (Z) erstrecken, wobei die Luftansaugleitung (1) und die Luftausstoßleitung (2) den kastenförmigen Gehäusekörper (31) am unteren Abschnitt (31a) bzw. am oberen Abschnitt (31b) gemäß einem im Wesentlichen tangentialen Winkel verbinden, wobei die röhrenförmige Kammer (7) durch eine Hülse gebildet wird, die in dem unteren Abschnitt (31a) untergebracht ist, so dass das zweite Ende (7c) der röhrenförmigen Kammer (7) an den oberen Abschnitt (31b) angrenzt, wobei der untere Abschnitt (31a) an seiner Basis durch eine durchlässige Membran (32) verschlossen ist, die die Vernebelungsmittel (9) trägt, wobei die durchlässige Membran (32) das Innere des kastenförmigen Gehäusekörpers (31) von dem Wasserbehälter (8) trennt, wobei der untere Abschnitt (31a) einen unteren torusförmigen Kanal (33) bildet, um die von der Luftansaugleitung (1) zugeführte Luft um die Basis der Hülse herum und in Richtung des Inneren der röhrenförmigen Kammer (7) über einen Durchgang zwischen dem ersten Ende (7b) der röhrenförmigen Kammer (7) und der durchlässigen Membran (32) zu verteilen, wobei der obere Abschnitt (31b) die Energieversorgungsmittel (6) trägt, das Laufradelement (4) zentral aufnimmt und um dieses herum einen oberen torusförmigen Kanal (35) für die radiale Verteilung der Luft und deren Zuführung zur Luftausstoßleitung (2) bildet.

15. Vorrichtung nach Anspruch 14, sofern dieser von Anspruch 7 abhängig ist, wobei die durchlässige Membran (32) eine Verteilung von Sitzen (32c) zum Aufnehmen der einen oder mehreren Folien (9) und eine Verteilung von Löchern (32d, 32f, 32 g, 32h) für die Kapillarkanäle (37) und für Auslassrohre (36) aufweist, die sich von dem ersten Ende (7b) der Hülse erstrecken, wobei das erste Ende (7b) der Hülse nach innen gefaltet ist, um eine periphere Rinne (7d) zu bilden, die dazu geeignet ist, mit Verunreinigungen vermischtes Wasser aufzufangen, das entlang der inneren Seitenfläche (7a) der röhrenförmigen Kammer (7) abfällt.

16. Vorrichtung nach Anspruch 12, wobei die röhrenförmige Kammer (7) so angeordnet ist, dass die Kammerachse (Z') parallel zur allgemeinen Erstreckungsebene (X', Y') der Vorrichtung verläuft oder auf dieser liegt.

17. Vorrichtung nach Anspruch 16, sofern abhängig von Anspruch 4, wobei sich zwischen dem Auslass (101b) des Ansaugkanals (10') und dem ersten Ende (107b) der röhrenförmigen Kammer (107) ein röhrenförmiger Kammereinlassverteiler (131) befindet, wobei der Innendurchmesser des röhrenförmigen Kammereinlassverteilers (131) im Wesentlichen dem der röhrenförmigen Kammer (107) an dem ersten Ende (107b) entspricht, wobei an der Innenfläche des Verteilers Sitze (132) zum Aufnehmen entsprechender piezoelektrischer Folien (109) ausgebildet sind, denen Wasser durch die Kapillarkanäle (137, 137b, 137c) zugeführt wird, die sich zwischen den jeweiligen Verteilersitzen (132) und dem Wasserbehälter (108) erstrecken, wobei Letzterer als radiale Erweiterung des röhrenförmigen Kammereinlassverteilers (131) in einer unteren Zone ausgebildet ist.

18. Vorrichtung nach Anspruch 17, wobei die radiale Erweiterung, die den Wasserbehälter (108) bildet, an ihrem Boden einen Hohlraum (108a) aufweist, der das Auffangen der Schadstoffpartikel unterstützt.

19. Vorrichtung nach einem der Ansprüche 16 bis 18, wobei ein Kammerauslassverteiler (139) zwischen dem zweiten Ende (107c) der röhrenförmigen Kammer und dem Einlass (102b) der Luftausstoßleitung (102) angeordnet ist und der Reihe nach, dem axialen Verlauf des pneumatischen Flusses folgend, ein zylindrisches Segment (139b) unmittelbar stromabwärts der röhrenförmigen Kammer (107) mit einem Durchmesser entsprechend dem des zweiten Endes (107c) der röhrenförmigen Kammer (107) und ein konisches Segment (139c) umfasst, das den Luftdurchgangsquerschnitt auf jenen der Luftausstoßleitung (102) verengt, wobei das zylindrische Segment (139b) mit der röhrenförmigen Kammer (107) mittels eines Verbindungsbechers (139a) verbunden ist, in dem das Zentrifugallaufradelement (104) untergebracht ist und das peripher einen torusförmigen Kanal (135a) zum Verteilen und Leiten der austretenden Luft stromabwärts vom Laufradelement (104) und daher zum zylindrischen Segment (139b) hin begrenzt, wobei in Letzterem ein Rotor (110) angeordnet ist, der zusammen mit dem Laufradelement (104) in Drehung versetzt wird und mit einer axialen Leitschaufel konfiguriert ist, um die Strömung aus dem torusförmigen Kanal (135a) in axialer Richtung weiter zu fördern und in einen ringförmigen Spalt (135b) einzuleiten, der noch innerhalb des zylindrischen Segments (139b) ein Gehäuse für die Energieversorgungsmittel (106) umgibt.

20. Vorrichtung nach einem der vorstehenden Ansprüche, wobei mindestens der Wasserbehälter (8) vom Rest der Vorrichtung abnehmbar getragen wird.

21. Vorrichtung nach einem der vorstehenden Ansprüche, wobei die Energieversorgungsmittel einen Motor (6) und wiederaufladbare Batteriemittel umfassen.

## Revendications

1. Dispositif portable de traitement d'air ayant une structure génériquement annulaire conçue pour être portée sur la tête (C) d'un utilisateur tout en laissant le visage de l'utilisateur essentiellement dégagé, le dispositif comprenant :
- au moins un conduit d'aspiration d'air (1), évoluant entre une entrée d'aspiration (1a) d'air à traiter contenant des particules polluantes en suspension, prévue pour être disposée près d'une région naso-buccale de l'utilisateur, et une sortie (1b) d'air prévue pour être disposée dans une région arrière de la tête de l'utilisateur (C) ;
- au moins un conduit d'éjection d'air (2) évoluant entre une sortie d'éjection d'air traité (2a) prévue pour être disposée près de la région naso-buccale de l'utilisateur et une entrée (2b) prévue pour être disposée dans la région arrière de la tête de l'utilisateur (C) ;
- une unité de traitement d'air (3) reliée auxdits conduits d'aspiration et d'éjection d'air (1, 2) et en communication pneumatique avec eux entre ladite sortie (1b) dudit conduit d'aspiration d'air (1) et ladite entrée (2b) dudit conduit d'éjection d'air (2), ladite unité de traitement d'air (3) étant prévue pour être disposée dans la région arrière de la tête de l'utilisateur (C) et comprenant : un moyen de propulsion de flux pneumatique depuis ledit conduit d'aspiration d'air (1) jusqu'audit conduit d'éjection d'air (2) ; des moyens de traitement d'air (5) conçus pour recevoir de l'air à traiter depuis ladite sortie (1b) dudit conduit d'aspiration d'air (1), et renvoyer de l'air traité vers ladite entrée (2b) dudit conduit d'éjection d'air (2) ; et des moyens d'alimentation (6) dudit moyen de propulsion de flux pneumatique et dudit moyen de traitement d'air (5) ;
dans lequel lesdits moyens de traitement d'air (5) comprennent :
- une chambre tubulaire (7) définissant une surface latérale intérieure (7a) de la chambre tubulaire (7) et évoluant le long d'un axe (Z) de la chambre tubulaire (7) entre une première extrémité (7b), de plus petit diamètre et en communication pneumatique avec ladite sortie (1b) du conduit d'aspiration d'air (1), et une seconde extrémité (7c), de plus grand diamètre, en communication pneumatique avec ladite entrée (2b) dudit conduit d'éjection d'air (2) ;
- des moyens de direction de flux pneumatique conçus pour favoriser une circulation tourbillonnaire à l'intérieur de ladite chambre tubulaire (7) ;
- au moins un réservoir d'eau (8) communiquant avec ladite chambre tubulaire (7) et placé près de ladite première extrémité (7b) ;
- un moyen de nébulisation (9) associé audit réservoir d'eau (8), conçu pour favoriser une émission d'eau nébulisée entrant dans ladite chambre tubulaire (7) par ladite première extrémité (7b),
de telle manière que :
ladite émission d'eau nébulisée est investie par l'air à traiter entrant dans ladite chambre tubulaire (7) à partir de ladite première extrémité (7b), et sous l'effet de ladite circulation tourbillonnaire un dépôt d'eau retenant lesdites particules polluantes se forme en adhérence sur ladite surface latérale intérieure (7a) de ladite chambre tubulaire (7) et tend à retourner dans ledit réservoir d'eau (8).

2. Dispositif selon la revendication 1, dans lequel lesdits moyens de propulsion de flux pneumatique comprennent au moins un élément de turbine (4).

3. Dispositif selon la revendication 2, dans lequel lesdits moyens de direction de flux pneumatique sont formés par ledit au moins un élément de turbine (4), conçu comme une turbine centrifuge et supporté coaxialement à l'intérieur de ladite chambre tubulaire (7).

4. Dispositif selon l'une quelconque des revendications précédentes, dans lequel ladite chambre tubulaire (7) a une forme tronconique.

5. Dispositif selon l'une quelconque des revendications précédentes, dans lequel lesdits moyens de nébulisation (9) comprennent des moyens d'agitation d'eau dans ledit au moins un réservoir d'eau (8).

6. Dispositif selon la revendication 5, dans lequel lesdits moyens d'agitation d'eau comprennent un ou plusieurs éléments piézoélectriques (9).

7. Dispositif selon la revendication 6, dans lequel lesdits un ou plusieurs éléments piézoélectriques comprennent une ou plusieurs feuilles (9) constituées de matériau poreux, lesdits moyens de nébulisation comprenant en outre des conduits capillaires (37) s'étendant entre ledit réservoir d'eau (8) et lesdites feuilles (9) pour acheminer de l'eau dudit réservoir (8) vers lesdites feuilles (9).

8. Dispositif selon la revendication 7, dans lequel lesdits conduits capillaires (37) sont assurés par un ou plusieurs corps constitués de matériau poreux ou spongieux.

9. Dispositif selon l'une quelconque des revendications précédentes, comprenant une ou plusieurs DEL à rayonnement UVC prévues pour exercer une action germicide sur de l'eau au moins dans ledit réservoir d'eau (8).

10. Dispositif selon la revendication 6, comprenant au moins une première bande DEL (38a) disposée au niveau dudit réservoir et une seconde bande DEL hélicoïdale (38b) évoluant autour de ladite chambre (7).

11. Dispositif selon l'une quelconque des revendications précédentes, dans lequel lesdits conduits d'aspiration d'air et d'éjection d'air (1, 2) comprennent une ou plusieurs sections élastiques, déformables ou articulées pour améliorer l'adaptation à la tête (C) de l'utilisateur.

12. Dispositif selon l'une quelconque des revendications précédentes, dans lequel ledit conduit d'aspiration d'air (1) et ledit conduit d'éjection d'air (2) se trouvent sensiblement au-dessus d'un plan de développement général (X, Y) du dispositif, qui, dans la position d'utilisation du dispositif, correspond à un plan transversal de la tête de l'utilisateur (C).

13. Dispositif selon la revendication 12, dans lequel ladite chambre tubulaire (7) est disposée avec ledit axe de chambre (Z) orthogonal au plan de développement général (X, Y) du dispositif.

14. Dispositif selon la revendication 13, prise en dépendance de la revendication 3, dans lequel ladite unité de traitement (3) comprend un corps de boîtier en forme de boîte (31) défini par une partie inférieure (31a) et une partie supérieure (31b), toutes deux en forme de disque générique et évoluant avec une symétrie sensiblement polaire par rapport audit axe de chambre (Z), lesdits conduits d'aspiration d'air (1) et d'éjection d'air (2) rejoignant ledit corps de boîtier en forme de boîte (31) respectivement au niveau de ladite partie inférieure (31a) et de ladite partie supérieure (31b) selon un angle sensiblement tangentiel, ladite chambre tubulaire (7) étant formée par un manchon logé dans ladite partie inférieure (31a) de sorte que ladite seconde extrémité (7c) de la chambre tubulaire (7) est adjacente à ladite partie supérieure (31b), ladite partie inférieure (31a) étant fermée à sa base par une membrane perméable (32) supportant ledit moyen de nébulisation (9), ladite membrane perméable (32) séparant l'intérieur dudit corps de boîtier en forme de boîte (31) dudit réservoir d'eau (8), ladite partie inférieure (31a) formant un canal toroïdal inférieur (33) pour la distribution d'air, alimenté par le conduit d'aspiration d'air (1), autour de la base du manchon et vers l'intérieur de la chambre tubulaire (7) à travers un passage entre la première extrémité (7b) de la chambre tubulaire (7) et ladite membrane perméable (32), ladite partie supérieure (31b) supportant ledit moyen d'alimentation (6), logeant au centre ledit élément de turbine (4) et formant autour de lui un canal toroïdal supérieur (35) pour la distribution radiale de l'air et l'acheminant vers le conduit d'éjection d'air (2).

15. Dispositif selon la revendication 14, prise en dépendance de la revendication 7, dans lequel ladite membrane perméable (32) fournit une distribution de sièges (32c) pour loger lesdites une ou plusieurs feuilles (9), et une distribution de trous (32d, 32f, 32 g, 32h) pour lesdits conduits capillaires (37) et pour des tubes de décharge (36) s'étendant à partir de la première extrémité (7b) du manchon, ladite première extrémité (7b) du manchon étant repliée vers l'intérieur pour former une gouttière périphérique (7d) adaptée pour collecter de l'eau mélangée à des impuretés tombant le long de la surface latérale intérieure (7a) de la chambre tubulaire (7).

16. Dispositif selon la revendication 12, dans lequel ladite chambre tubulaire (7) est disposée avec ledit axe de chambre (Z') parallèle à, ou reposant sur, ledit plan de développement général (X', Y') du dispositif.

17. Dispositif selon la revendication 16 prise en dépendance de la revendication 4, dans lequel un collecteur tubulaire d'admission de chambre (131) entre ladite sortie (101b) du canal d'aspiration (10') et ladite première extrémité (107b) de la chambre tubulaire (107), ledit collecteur tubulaire d'admission de chambre (131) ayant un diamètre intérieur correspondant sensiblement à celui de la chambre tubulaire (107) au niveau de la première extrémité (107b), sur la surface intérieure de sièges de collecteur (132) étant formés pour loger des feuilles piézoélectriques respectives (109), alimentées en eau par lesdits conduits capillaires (137, 137b, 137c) s'étendant entre des sièges de collecteur respectifs (132) et ledit réservoir d'eau (108), ce dernier étant conçu comme une expansion radiale du collecteur tubulaire d'admission de chambre (131) au niveau d'une zone inférieure.

18. Dispositif selon la revendication 17, dans lequel ladite expansion radiale formant ledit réservoir d'eau (108) comporte à sa base une cavité (108a) aidant à la collecte des particules polluantes.

19. Dispositif selon l'une quelconque des revendications 16 à 18, dans lequel un collecteur de sortie de chambre (139) est disposé entre la seconde extrémité (107c) de la chambre tubulaire et ladite entrée (102b) du conduit d'éjection d'air (102), et comprend, en suivant successivement le parcours axial du flux pneumatique, un segment cylindrique (139b) immédiatement en aval de la chambre tubulaire (107), ayant un diamètre correspondant à celui de ladite seconde extrémité (107c) de la chambre tubulaire (107), et un segment conique (139c) qui rétrécit la section de passage d'air jusqu'à celle du conduit d'éjection d'air (102), ledit segment cylindrique (139b) étant relié à la chambre tubulaire (107) au moyen d'une coupelle de raccordement (139a) dans laquelle est logé ledit élément de turbine centrifuge (104) et qui définit à la périphérie un canal toroïdal (135a) pour distribuer et diriger l'air sortant, en aval dudit élément de turbine (104), et donc vers le segment cylindrique (139b), dans ce dernier un rotor (110) étant disposé, entraîné en rotation avec ledit élément de turbine (104) et conçu avec une pale axiale pour favoriser davantage l'écoulement dudit canal toroïdal (135a) dans la direction axiale, et pour l'introduire dans un espace annulaire (135b) qui toujours à l'intérieur du segment cylindrique (139b) entoure un logement pour lesdits moyens d'alimentation (106).

20. Dispositif selon l'une quelconque des revendications précédentes, dans lequel au moins ledit réservoir d'eau (8) est supporté de manière amovible du reste du dispositif.

21. Dispositif selon l'une quelconque des revendications précédentes, dans lequel lesdits moyens d'alimentation comprennent un moteur (6) et des moyens de batterie rechargeable.
